# EUROPEAN PATENT APPLICATION

(11) **EP 4 306 117 A1**
(43) Date of publication of application: **17.01.2024**
(21) Application number: 22185533.1
(22) Date of filing: 18.07.2022
(51) Int. Cl.: A61K 35/12, A61K 47/00, C12N 1/36, C12N 5/071, C12N 5/078

(54) **BIOMATERIAL FOR IMMUNOMODULATION, PROCESS FOR ITS PRODUCTION AND USES THEREOF**

(30) Priority: 13.07.2022 PT 2022118103
(71) Applicant: Universidade de Aveiro, 3810-193 Aveiro (PT)
(72) Inventor: COLARDELLE DA LUZ MANO, JOÃO FILIPE, 3810-202 AVEIRO (PT); BRAGA DE OLIVEIRA, MARIANA, 3800-260 AVEIRO (PT); FORTUNATO SOUSA, ANA RITA, 2460-790 TURQUEL (PT)
(74) Representative: Monteiro Alves, Inês

(57) **Abstract**

The present invention provides a biomaterial for immunomodulation comprising aggregates of phenotypically stabilized cells. Said biomaterial is meant to be preferentially administered in a limited location and provides immunomodulation for preventive or therapeutic purposes.

It is also part of the present invention the process for production of the mentioned biomaterial and uses thereof. More specifically, the biomaterial for immunomodulation herein disclosed adds benefit in contexts of transplantation, chronic inflammation, autoimmune diseases, acute inflammation, cancer treatment, or in injured tissue regeneration.

## Description

### TECHNICAL FIELD

The present invention relates to cell-based therapies and provides a biomaterial for immunomodulation comprising aggregates of phenotypically stabilized cells, the process for its production and uses thereof.

Said biomaterial is meant to be preferentially administered in a limited location and provides immunomodulation for preventive or therapeutic purposes.

### BACKGROUND OF THE INVENTION

Cell-based therapeutics have enabled controlling the host's microenvironment and immune system. Such cellular therapies may exhibit immunomodulatory features which may either activate or suppress the immune system's function.

Cell-based immunomodulatory technologies have been proposed as strategies to replace or reduce the use of classical systemically administered and non-specific immunosuppressants in contexts of chronic inflammation and transplant rejection (Woodward, K.B. et al., 2020). Those may also find application as anti-inflammatory and pro-healing agents for the regeneration of injured tissues, or even as activators of the immune system to treat cancer or infections (Lin, J.C. et al., 2021).

The treatment of most immune-mediated pathologies, such as chronic inflammatory diseases or conditions, and the prevention of transplant rejection, relies on the prolonged (or lifelong) use of systemic and nontargeted immunosuppressants as the gold standard (Wekerle, T. et al., 2017). The subsequent global immunodepression of patients by the constant exposure to systemic immunosuppressant drugs predisposes them to infections and to the development of malignant tumors. These severe long-term morbidities most times invalidate the therapeutic effect of transplants (Sellares, J. et al., 2012).

Several cell-based therapies were suggested to reduce or replace the use of classical immunosuppressants and anti-inflammatory drugs in inflammation settings (Sousa, A.R. et al., 2021). Those are based on strategies such as i) the adoptive transfer of tolerogenic cells; ii) the delivery of cells artificially engineered towards tolerogenicity; iii) approaches combining immunomodulatory cells and biomaterials; and iv) the induction of hematopoietic chimerism regimens (WO2016/086020A1; Kobayashi, S. et al., 2020; Sawitzki, B. et al., 2020; Mao, A.S. et al., 2019; and Duran-Struuck, R. et al., 2017).

The administration and retention of immunomodulatory therapeutics in a specific location has been explored as a way to decrease the intake of systemic immunosuppressants to minimal administration regimens, while locally controlling phenomena as graft rejection (Woodward, K.B. et al., 2020), or achieving localized tissue regeneration and vascularization (Dellacherie, M.O. et al., 2019).

In particular, biomaterial-based strategies comprising the presentation of specific domains present in cells have been used to provide localized immunomodulation in the context of allogeneic transplantation (US6875430B2; Woodward, K.B. et al., 2020; and Headen, D.M. et al., 2018).

There are few reports concerning systems capable of providing immunomodulatory activity in a spatially defined manner, using exogenous biomaterials (Headen, D.M. et al. (2018); and Garcia, J.R. et al. (2019)), and mostly targeting tissue regeneration.

The premises above exposed characterize the technical problem that in the present disclosure relates to create an immunomodulatory cell-based technology to replace or reduce the use of non-specific immunosuppressants in context of any inflammatory disease or condition, such as in transplantation, for instance, and also trigger suppression of acute inflammation scenarios and its symptoms, for example benefiting several regenerative medicine applications.

It has been strongly suggested that the effectiveness of therapies towards chronic conditions (e.g., osteoarthritis) depends on a long-lasting effect of immunomodulatory effects (Scanzello, C.R., 2017).

Living cells have the ability to change their phenotype when exposed to different environmental cues, and also undergo through death (e.g., apoptosis) in a certain period of time (Galleu et al., 2017; and Nombela-Arrieta, C. et al., 2011).

However, there are no reports of cell-based systems capable of providing long-lasting and stable immunomodulatory activity in a localized manner.

Therefore, the present invention provides a biomaterial for immunomodulation, which comprises aggregates of phenotypically stabilized cells, preferably devitalized, with preferential use in the localized administration and prolonged treatment of inflammatory diseases or conditions, and for regenerative medicine.

It is also expected that the fine tuning / tailoring of the features of the present biomaterial promotes the activation of the immune system. Therefore, such immunogenic tailoring may benefit any conditions or diseases where the activation of the immune system function is desired, for example, in the treatment / diagnosis of neoplasms, and to treat bacterial and viral infections.

### TECHNICAL PROBLEM

Cell-based therapies holds enormous promise for treating many different diseases. However, the ability of cells to move from placement sites, change into inappropriate cell types or multiply affect the effectiveness of the therapy due to failure of cells to work as expected.

### SOLUTION TO PROBLEM

The present invention provides a biomaterial for immunomodulation comprising aggregates of phenotypically stabilized cells. Said biomaterial is meant to be preferentially administered in a limited location and provide potential to reduce or replace the use of classical immunosuppressants and anti-inflammatory drugs in context of inflammatory diseases or conditions, as well as for regenerative medicine.

### SUMMARY OF THE INVENTION

In one aspect of the invention, it is disclosed a biomaterial for immunomodulation comprising:
- 60% to 100% of aggregates of phenotypically stabilized cells or parts thereof, in dimension ranges of 100 µm to 1 m; and
- 0% to 40% of added biomaterial components, in dimension ranges of 1 µm to 500 µm;

wherein the added biomaterial components are selected from the group consisting of cell-derived materials and exogenously provided materials selected from the group consisting of natural polymers, ceramics, glasses and synthetic polymers;
wherein the cells or parts thereof are natural, engineered, or primed eukaryotic or non-eukaryotic cells or parts thereof or combinations of eukaryotic cells, non-eukaryotic cells, or parts thereof, provided that the eukaryotic cells, non-eukaryotic cells, or parts thereof have immunomodulatory activity; and
wherein the added biomaterial components and / or the aggregates of phenotypically stabilized cells or parts thereof are joined together through physical forces and / or covalent or non-covalent chemical bonds.

In a second aspect of the invention, it is disclosed a process for production of the biomaterial for immunomodulation comprising the steps of:
a) tailoring the cells to the desired phenotype;
b) adjusting culture conditions to promote the formation of the cells aggregates; and
c) phenotypically stabilizing the aggregates.

In a third aspect of the invention, it is disclosed the use of the biomaterial for immunomodulation in the treatment, diagnosis and / or prevention of complications in acute and chronic inflammatory diseases or conditions, immune-mediated diseases or conditions, transplantation and post-transplantation of allogeneic grafts or full organs, regenerative medicine, tumors, and infections caused by pathogens such as bacteria, mycobacteria or virus.

### BRIEF DESCRIPTION OF THE FIGURES

In order to promote an understanding of the principles according to the modalities of the present invention, reference will be made to the embodiments illustrated in the figures and the language used to describe them.

It should also be understood that there is no intention to limit the scope of the invention to the content of the figures. The following figures provide preferred embodiments for illustrating the description and should not be seen as limiting the scope of disclosure. Modifications to the inventive features illustrated herein, as well as additional applications of the principles and embodiments illustrated, which would normally occur to a person skilled in the art having the possession of this description, are considered within the scope of the claimed invention.

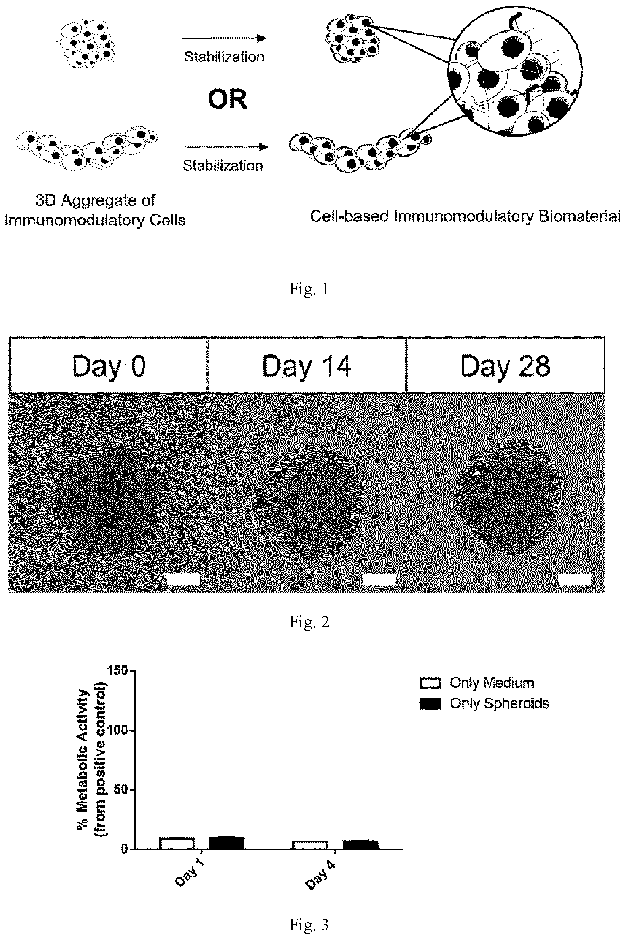

Figure 1: Schematic representation of the process for production of the cell-based biomaterial of the present invention.
Figure 2: Morphology of cell aggregates prepared from human mesenchymal stem cells derived from the adipose tissue after phenotypic stabilization, in 0, 14 and 28 days of incubation in DPBS, at 37 °C. Scale bar: 50 µm.
Figure 3: Quantification of the cellular metabolic activity (MTS assay) of the phenotypically stabilized spheroids (2000 cells / spheroid; total of 10 or 20 spheroids) prepared from mesenchymal stem cells isolated from the adipose tissue, after 1 and 4 days of culture in cell culture medium; mean ± SD (n = 4 replicates).

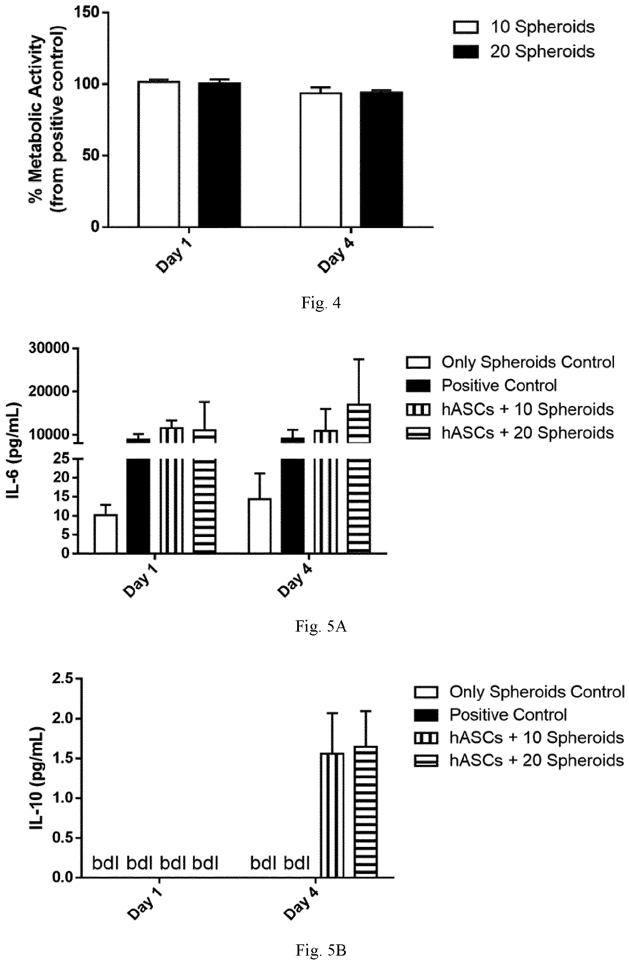

Figure 4: Quantification of cellular metabolic activity (MTS reduction assay) of human mesenchymal stem cells derived from adipose tissue cultured in a monolayer in the presence of different amounts of the phenotypically stabilized spheroids, after 1 and 4 days in cell culture; mean ± SD (n = 4 replicates).
Figure 5: Quantification of the secretion of (A) IL-6 and (B) IL-10 from the living monolayer of human mesenchymal stem cells derived from the adipose tissue, in the presence of different amounts of the phenotypically stabilized spheroids, after 1 and 4 days in cell culture; mean ± SD (n = 4 replicates).

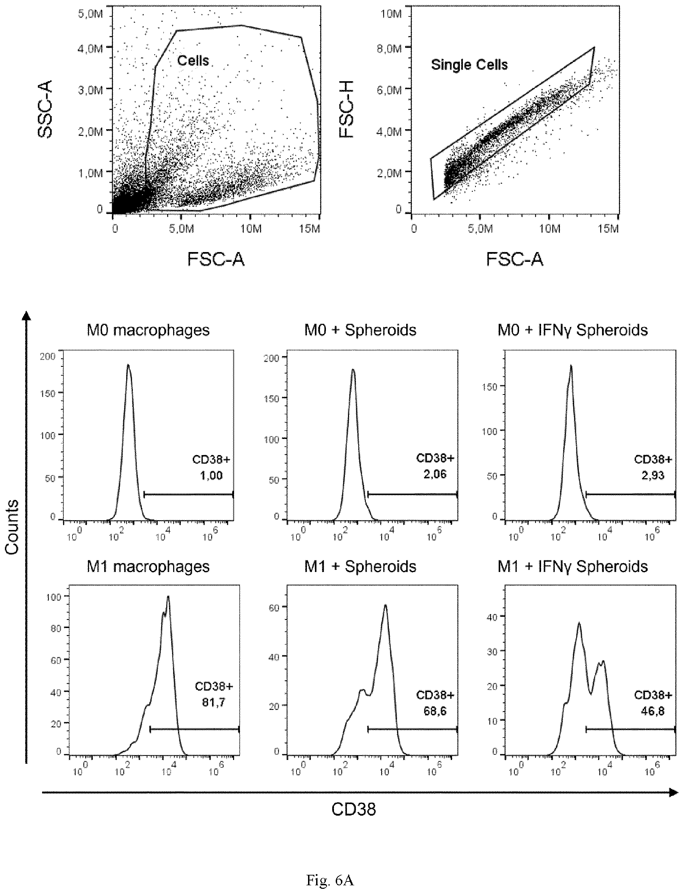

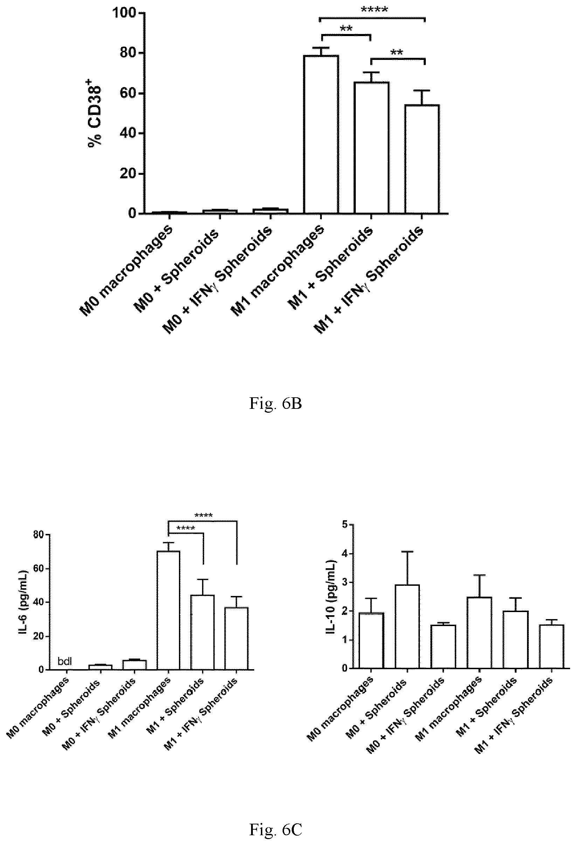

Figure 6: (A) Flow cytometry chart showcasing the gating strategy used to analyze the distribution of THP-1 macrophages in different polarization states (M0 and M1) and to establish the cell populations expressing the CD38 surface marker. (B) Quantification (flow cytometry) of the percentage of positive THP-1 macrophages for the CD38 surface marker; results for 50.000 THP-1 macrophages polarized towards M0 or M1 states in the presence of an average 6 phenotypically stabilized spheroids or IFNy-primed spheroids for 1 day of contact. (C) Quantification of the secretion of IL-6 and IL-10 by THP-1 macrophage cells in the presence of an average 6 phenotypically stabilized spheroids or IFNy-primed spheroids; mean ± standard deviation (n = 4 replicates). Statistical significance determined using One-way ANOVA, Tukey's multiple comparisons test.

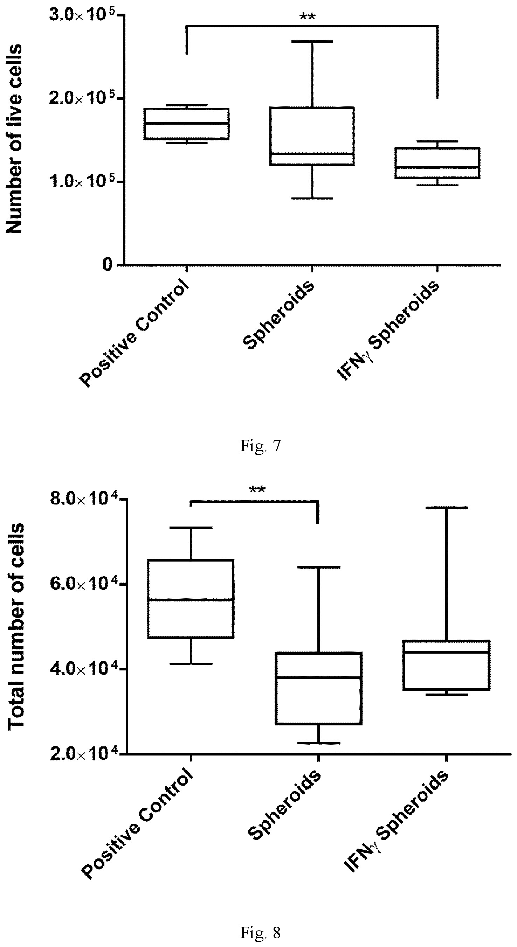

Figure 7: Quantification of live cells (Trypan blue assay) in a population of primary human CD4+ T lymphocytes activated with CD3/CD28 magnetic beads. Results for 100,000 pre-activated T cells in the presence of 8 phenotypically stabilized spheroids or IFNy-primed spheroids for 4 days of contact; median ± min/max (n = at least 4 replicates). Statistical significance determined using unpaired t test.
Figure 8: Quantification of total number of cells in a population of primary human CD8+ T lymphocytes activated with CD3/CD28 magnetic beads. Results for 100,000 pre-activated T cells in the presence of 8 phenotypically stabilized spheroids for 4 days of contact; median ± min/max (n = 8 replicates). Statistical significance determined using unpaired t test.

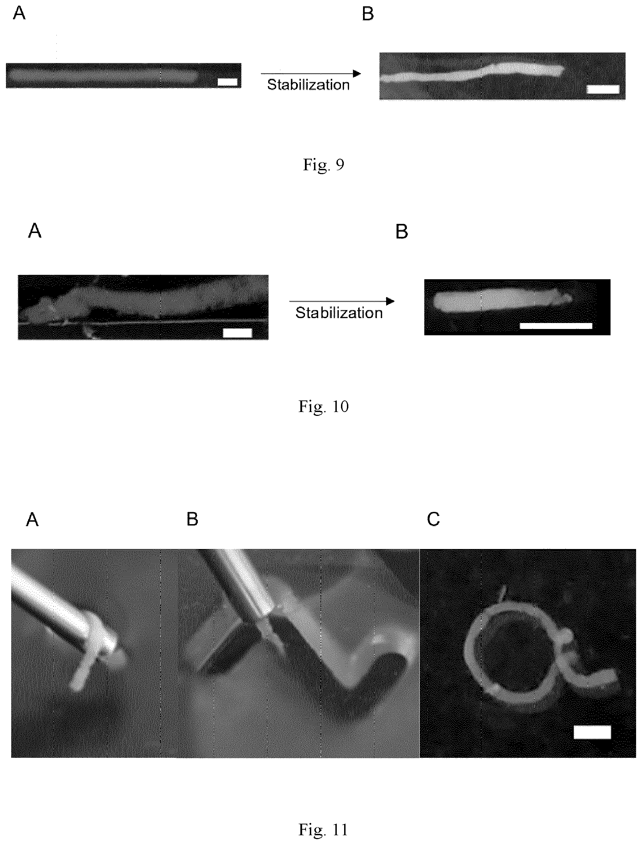

Figure 9: Image of a cell fiber prepared from mesenchymal stem cells isolated form the adipose tissue (1 million cells/fiber) using fetal bovine serum- supplemented medium, while (A) living and (B) after phenotypic stabilization. Scale bar: 2 mm.
Figure 10: Image of a cell fiber prepared from mesenchymal stem cells isolated from the adipose tissue (1 million cells/fiber) using human platelet lysates-supplemented medium, in xenogeneic factor-free conditions, while (A) living and (B) after phenotypic stabilization. Scale bar: 2 mm.
Figure 11: Image of a phenotypically stabilized cell fiber prepared from mesenchymal stem cells isolated from the adipose tissue using human platelet lysates-supplemented medium, in xenogeneic factor-free conditions, highlighting a malleable behavior, while (A) easily handled by needle, (B) adapting to a N-shaped defect and (C) folded in an 'a' shape; scale bar: 1 mm.

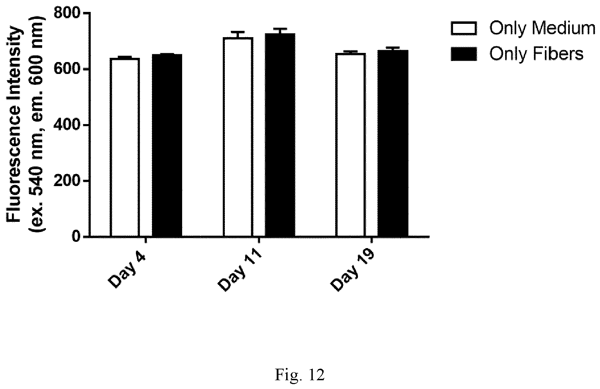

Figure 12: Metabolic activity quantification (AlamarBlue^{™} test) of phenotypically stabilized cell fiber prepared from mesenchymal stem cells isolated from the adipose tissue using human platelet lysates-supplemented medium, in xenogeneic factor-free conditions, after 4, 11 and 19 days of culture in cell culture standard conditions, showcasing the effective phenotypically stabilized of a larger in vitro-prepared tissue; mean ± standard deviation (n = 6 replicates).

### DETAILED DESCRIPTION OF THE INVENTION

The present invention discloses a biomaterial for immunomodulation comprising:
- 60% to 100% of aggregates of phenotypically stabilized cells or parts thereof, in dimension ranges of 100 µm to 1 m; and
- 0% to 40% of added biomaterial components, in dimension ranges of 1 µm to 500 µm;

wherein the added biomaterial components are selected from the group consisting of cell-derived materials and exogenously provided materials selected from the group consisting of natural polymers, ceramics, glasses and synthetic polymers;
wherein the cells or parts thereof are natural, engineered, or primed eukaryotic or non-eukaryotic cells or parts thereof or combinations of eukaryotic cells, non-eukaryotic cells, or parts thereof, provided that the eukaryotic cells, non-eukaryotic cells, or parts thereof have immunomodulatory activity; and
wherein the added biomaterial components and / or the aggregates of phenotypically stabilized cells or parts thereof are joined together through physical forces and / or covalent or non-covalent chemical bonds.

By part of the cells, it is meant the cell's components, as well as their extracellular content, including cell-derived materials, such as extracellular matrix components and excreted vesicles (e.g., exosomes, extracellular vesicles), or other soluble molecules.

In a preferred embodiment of the invention, the biomaterial for immunomodulation of the present invention has long-lasting control over its three-dimensional structure and architecture. More specifically, the biomaterial for immunomodulation has a reconfigurable shape which allows the biomaterial to adapt to defects with complex shapes. Therefore, the biomaterial has a shape selected from the group consisting of spheres, fibers, polyhedrons, cell-sheets, bioinks, cylinders, foams, hydrogels, sponges, reticulated cell fragments obtained through disintegration of phenotypically stabilized cell constructs (for example, through mechanical forces including cutting), cream, ointments, patches, and adhesives.

Biomaterial components may be added to the biomaterial for immunomodulation of the present invention as a conditioning to promote specific cell phenotypes or impart desired mechanical properties.

The added biomaterial components are selected from the group consisting of cell-derived materials and exogenously provided materials selected from the group consisting of natural polymers, ceramics, glasses, and synthetic polymers.

In a preferred embodiment of the invention, cell-derived materials are extracellular matrix components and excreted vesicles (e.g., exosomes, extracellular vesicles), as well as other soluble molecules. The exogenously provided materials includes natural polymers (e.g., alginate, chitosan, dextran, collagen, gelatin, and recombinant peptides), ceramics and glasses (e.g., hydroxyapatite, calcium phosphates and bioactive glasses) and synthetic polymers (e.g., polyethylene glycol, poly (lactic-co-glycolic acid), poly(caprolactone), polystyrene.

The added biomaterial components may have any shape selected from spherical or polyhedral or combinations thereof and the cell aggregates are shaped polyhedrons selected from cells spheroids, cell fibers, cell monolayers, or cells multilayers. The biomaterial components and the cell aggregates are joined together through physical forces (e.g., centrifugation, gravity) and / or covalent or non-covalent chemical bonds (e.g., hydrogen bonds, electrostatic interactions, or formation of Schiff bases) to generate structures with larger dimensions.

In a preferred embodiment of the invention, the cell aggregates are spherical cell aggregates with 50 to 2000 µm diameter, prepared using 10 to 1 million cells, or fiber-shaped aggregates, deposited in any direction, with length from 200 µm to 1 m.

The cell types or parts thereof have immunomodulatory activity or are artificially engineered towards immunomodulation.

The eukaryotic cells may include, but are not limited to: mesenchymal stem cells, chimeric-antigen receptor T cells (CAR T-cells), regulatory T-cells (Tregs), dendritic cells, macrophages, lymphocytes such as T lymphocytes, B lymphocytes, NK lymphocytes, NKT lymphocytes, cells from adaptive and innate immune system and non-hematopoietic cells that crosstalk with immune system, such as hepatocytes, hepatic stellate cells, liver sinusoidal endothelial cells, and combinations thereof. In a preferred embodiment of the present invention, the cells are human mesenchymal stem cells isolated from the adipose tissue.

The eukaryotic cells or parts thereof have immunomodulatory activity through mechanisms that may comprise the presentation of immunomodulatory molecules from cells or extracellular matrix, the release of molecules, vesicles or fragments from the biomaterial, or the release of parts of the biomaterial through naturally occurring degradation or cell-mediated degradation (e.g., via phagocytic activity).

The non-eukaryotic cell types are selected from the group consisting of bacteria, cyanobacteria, and archaea.

In a preferred embodiment of the present invention, the biomaterial for immunomodulation further comprises at least one immunomodulatory molecule selected from the group consisting of proteins or glycosaminoglycans from cells or cell-derived components as vesicles or the extracellular matrix, which may include an antigen, an engineered antigen, an antigen presented in the context of major histocompatibility complex molecules (MHC, or human leucocyte antigen, HLA) or engineered forms thereof, MHC/HLA molecules or engineered forms thereof, co-stimulation molecules, cluster of differentiation 80/86 (CD80/CD86), Fas ligand (Fas-L), programmed death-ligand 1 (PD-L1), HLA class I (HLA-I), HLA-II, CTLA-4 inhibitors, PD1/PD-L1 inhibitors, Fas inhibitors, the clusters of differentiation CD3 or CD28, co-stimulation blockade molecules; immune checkpoint inhibitors, soluble or engineered soluble molecules; transmembrane or engineered transmembrane molecules and membrane or engineered membrane molecules and combinations thereof.

Such immunomodulatory molecule is naturally present in the biomaterial, deriving from the cell aggregate; or it is artificially added to the biomaterial using non-covalent or covalent bonds (for example, through adsorption processes or using chemical crosslinkers such as genipin, glutaraldehyde, EDC/NHS); or it is present in the biomaterial due to the previous stimulation of cells, where cells are stimulated to produce the molecules of interest, through artificial exposure of cells to immunomodulatory, chemical and physical factors or genetic engineering tools.

Chemical factors are selected from temporary anoxia, hypoxia - 0.5% to 10% O₂ - or hyperoxia, while physical factors are selected from shear stress and contact with biomaterial microparticles. Genetic engineering tools are selected from the group consisting of gene addition or removal technologies, gene editing technologies and gene replacement technologies. Other tools include for example the post-translational protein depletion and RNA engineering.

Another aspect of the present invention discloses the process for production of the biomaterial for immunomodulation, wherein the process comprises the steps of:
a) tailoring the cells to the desired phenotype;
b) adjusting culture conditions to promote the formation of the cells aggregates;
   and
c) phenotypically stabilizing the aggregates.

In step a), the cells are incubated and exposed to immunomodulatory, chemical, and physical factors and / or exposed to immunomodulatory agents selected from the group consisting of pro- or anti-inflammatory cytokines, such as IFNy, TNF-α, IL-1β, IL-4, IL-10.

Chemical factors are selected from different oxygen concentrations (such as temporary anoxia, hypoxia - 0.5% to 10% O₂ - or hyperoxia) and physical factors are selected from mechanical stimulation (such as shear stress provided by stirring tank bioreactors, hydrostatic pressure, or combinations thereof) and pH, temperature, and pressure variations.

The fine tuning of the features of the biomaterial of the present invention promotes the activation or the suppression of the immune system, benefiting any conditions or diseases where the activation or the suppression of the immune system function is desired, respectively.

After tailoring the cells, in step b), the cells are aggregated while in incubation in cell culture medium supplemented with fetal bovine serum or xenogeneic-free factors, such as human platelet lysates, and antibiotic / antimycotic solution. In an alternative embodiment, the cells are aggregated while in incubation in cell culture medium supplemented with immunomodulatory agents, added biomaterial components, chemical, or physical factors.

In a preferred embodiment of the invention, the cell culture medium is selected from alpha-MEM, DMEM, RPMI 1640.

The immunomodulatory agents, added biomaterial components, chemical, or physical factors are the same as previously disclosed.

The aggregates ensure the prolonged presentation of cell surface molecules and extracellular content overtime, while releasing soluble molecules and insoluble components of immunomodulatory and pro-regenerative interest.

In step c), the stabilization of the aggregates is performed by the inhibition of the cellular metabolic activity or the turnover of molecules, by enucleation, or by cellular devitalization and the cell aggregates are immersed in an organic solvent for an exposure period that varies from 1 minute to 96 hours, preferably 24 hours, and a temperature range that varies from 4°C to 120°C.

The organic solvent is selected from the group consisting of ethanol, methanol, dichloromethane, tetrahydrofuran, and chloroform. Other agents, such as cell fixating agents (e.g., formaldehyde, paraformaldehyde, glutaraldehyde, genipin, EDC/NHS), may be used during the stabilization step.

In alternative embodiments, the stabilization step is performed by any other method that inhibits cellular metabolic activity and the turnover of molecules, by enucleation or by cellular devitalization such as, incubation in hyper- or hypotonic saline solutions (e.g., NaCl), incubation in high acidic or alkaline solutions (e.g., concentrated solutions of HCl or NaOH), hydrogelation, lyophilization, supercritical drying, critical point drying, and combinations thereof.

At the end of the process, the cell aggregates can be used directly as the biomaterial for immunomodulation, or the cell aggregates can be shaped as desired to adapt to defects with complex shapes. It can be stored between -80 °C and 37 °C.

The biomaterial for immunomodulation of the present invention is useful as an autologous, allogeneic, or xenogeneic approach in the treatment, diagnosis and / or prevention of complications in acute and chronic inflammatory diseases or conditions, immune-mediated diseases or conditions, transplantation and post-transplantation of allogeneic grafts or full organs, regenerative medicine, tumors, and infections caused by pathogens such as bacteria, mycobacteria, or virus.

The cells comprised in the biomaterial provide interaction with the surrounding elements to mimic the native immune synapse, activating or suppressing the immune system and being useful in human or animal autologous, allogeneic, or xenogeneic approaches.

The biomaterial can be used synergistically with immunosuppressant drugs, including but not restricted to rapamicyn, cyclosporin, or anti-inflammatory drugs.

### EXAMPLES OF THE INVENTION

The cells comprised in the biomaterial for immunomodulation of the present invention must present, at the same time, the capability of:
- maintaining a three-dimensional structure overtime in physiological saline solutions;
- showing a stable phenotype overtime;
- not displaying cytotoxicity towards animal cells; and
- inducing an immunomodulatory action towards innate immune cells and adaptive immune cells.

Also, it is essential that the biomaterial for immunomodulation of the present invention:
- is capable of being extrapolated to different shapes while maintaining phenotypic stability or produced with xenogeneic-free factors;
- do not induce a pro-inflammatory phenotype in innate immune cells such as macrophages;
- decreases the expression of pro-inflammatory markers in macrophages pre-polarized towards the M1 phenotype; and
- decreases the proliferation of activated human CD4⁺ and CD8⁺ T lymphocytes.

Due to this fact, several tests were performed.

To evaluate the maintenance of three-dimensional structure of the construct overtime in physiological saline solutions, cell aggregates made of human mesenchymal stem cells derived from the adipose tissue were produced in the shape of spheroids or fibers, followed by phenotypic stabilization (Figure 1).

The phenotypically stabilized cell spheroid aggregates made of human mesenchymal stem cells derived from the adipose tissue kept their morphology and three-dimensional structure for 28 days of incubation in DPBS at 37°C (Figure 2).

To evaluate the phenotypic stabilization of the constructs, in one example, the quantification of the cellular metabolic activity (MTS assay) was performed, where phenotypically stabilized cell spheroid aggregates made of human mesenchymal stem cells derived from the adipose tissue (2000 cells / spheroid; total of 10 or 20 spheroids) exhibited no metabolic activity after 1 and 4 days in alpha-MEM cell culture medium (Figure 3).

To evaluate the toxicity of the constructs, in one embodiment, it was performed the quantification of the cellular metabolic activity (MTS assay) of human mesenchymal stem cells derived from adipose tissue cultured in a monolayer in the presence of different amounts of phenotypically stabilized spheroids (10 or 20). The phenotypically stabilized spheroids did not induce toxicity after 1 and 4 days of contact (Figure 4).

In one embodiment, the construct was evaluated for their immunomodulatory activity towards mesenchymal stem cells. In the same embodiment, the immunomodulatory activity of the constructs was evaluated by quantification, through ELISA assay, of pro- and anti-inflammatory molecules (IL-6 and IL-10, respectively), secreted by mesenchymal stem cells derived from adipose tissue cultured in a monolayer in the presence of different amounts (10 or 20) of phenotypically stabilized spheroids. After 1 and 4 days of contact, the construct enabled the modulation of the secretion of IL-6 and IL-10 molecules (Figure 5A, 5B).

In one example, the construct was evaluated for their immunomodulatory activity towards macrophages. In the same example, the immunomodulatory activity of the constructs was evaluated by quantification of pro-inflammatory markers (CD38, through flow cytometry, Figure 6A, 6B) and pro- and anti-inflammatory cytokines (IL-6 and IL-10, respectively, through ELISA assay, Figure 6C) exhibited by human THP-1 macrophages in different polarization states (M0 and M1), when in direct contact with an average of 6 phenotypically stabilized spheroids or IFNy-primed spheroids for 1 day. The constructs did not induce a pro-inflammatory phenotype in human macrophages and further reduced the pro-inflammatory phenotype in human macrophages pre-polarized towards a pro-inflammatory phenotype, seen by the reduced pro-inflammatory markers CD38 and IL-6 (Figure 6A, 6B, 6C).

In one embodiment, the construct was evaluated for their immunomodulatory activity towards CD4⁺ T lymphocytes. In the same embodiment, the immunomodulatory activity of the constructs was evaluated by quantification of live cells through Trypan blue assay, where a population of primary human CD4⁺ T lymphocytes was pre-activated with CD3/CD28 magnetic beads and incubated with 8 phenotypically stabilized spheroids or IFNγ-primed spheroids for 4 days of contact. The construct reduced the proliferation of the population of activated human CD4⁺ T lymphocytes (Figure 7).

In one embodiment, the construct was evaluated for their immunomodulatory activity towards CD8⁺ T lymphocytes. In the same embodiment, the immunomodulatory activity of the constructs was evaluated by quantification of total number of cells, using a hemocytometer, where a population of primary human CD8⁺ T lymphocytes was pre-activated with CD3/CD28 magnetic beads and incubated with 8 phenotypically stabilized spheroids or IFNγ-pnmed spheroids for 4 days of contact. The construct reduced the proliferation of the population of activated human CD8⁺ T lymphocytes (Figure 8).

To evaluate the ability to extrapolate the cell-based technology to fiber-shaped structures and maintain their phenotypic stabilization, in one example, the cell aggregates made of human mesenchymal stem cells derived from the adipose tissue were shaped into fibers (1 million cells/fiber), using fetal bovine serum- supplemented medium (Figure 9).

In other example, the fiber-shaped structures (1 million cells/fiber) were produced with the xenogeneic-free cell medium supplementing factor human platelet lysates (Figure 10).

In other example, the cell fiber aggregates made of human mesenchymal stem cells derived from the adipose tissue and produced using fetal bovine serum-supplemented medium exhibited a malleable behavior, while easily handled by needle (Figure 11A), adapting to a *N*-shaped defect (Figure 11B), and folded in an 'a' shape (Figure 11C).

In one embodiment, the maintenance of phenotypic stabilization of the cell fiber aggregates produced using fetal bovine serum-supplemented medium was evaluated by the quantification of the cellular metabolic activity (AlamarBlue assay) at days 4, 11 and 19, showcasing the effective phenotypically stabilization of a larger in vitro-prepared tissue (Figure 12).

The subject matter described above is provided as an illustration of the present invention and, therefore, should not be construed to limit it. The terminology employed for the purpose of describing preferred embodiments of the present invention should not be restricted to them.

As used in the description, definite and indefinite articles, in their singular form, are intended for interpretation to also include plural forms, unless the context of the description explicitly indicates otherwise.

The term "one", as well as the articles "a" and "an", should generally be interpreted as "one or more" unless specified otherwise or clear from the context to be directed to a singular form.

It will be understood that the terms "comprise" and "include", when used in the present description, specify the presence of features, elements, components, steps, and related operations, but do not exclude the possibility of other features, elements, components, steps, and operations as well contemplated.

As used throughout the present patent application, the term "or" is used in an inclusive sense rather than an exclusive sense, unless the exclusive meaning is clearly defined in a specific situation. In this context, a phrase of the type "X uses A or B" should be interpreted as including all relevant inclusive combinations, for example "X uses A", "X uses B" and "X uses A and B".

All modifications, provided they do not change the essential features of the following claims, must be considered within the scope of the protection of the present invention.

### CITATION LIST

The citation list is as follows:

### PATENT LITERATURE

- WO2016/086020A1
- US6875430B2

### NON-PATENT LITERATURE

- Dellacherie, M.O. et al. (2019) Macroscale biomaterials strategies for local immunomodulation. Nature Reviews Materials 4 (6), 379-397.
- Duran-Struuck, R. et al. (2017) Effect of Ex Vivo-Expanded Recipient Regulatory T Cells on Hematopoietic Chimerism and Kidney Allograft Tolerance Across MHC Barriers in Cynomolgus Macaques. Transplantation 101 (2), 274-283.
- Galleu et al. (2017) Apoptosis in mesenchymal stromal cells induces in vivo recipient-mediated immunomodulation. Sci. Transl. Med. 9, eaam7828.
- Garcia, J.R. et al. (2019) IFN-gamma-tethered hydrogels enhance mesenchymal stem cell-based immunomodulation and promote tissue repair. Biomaterials 220, 119403.
- Headen, D.M. et al. (2018) Local immunomodulation with Fas ligand-engineered biomaterials achieves allogeneic islet graft acceptance. Nat Mater 17 (8), 732-739.
- Kobayashi, S. et al. (2020) A biomimetic five-module chimeric antigen receptor ((5M)CAR) designed to target and eliminate antigen-specific T cells. Proc Natl Acad Sci U S A 117 (46), 28950-28959.
- Lin, J.C. et al. (2021) Facile Transformation of Murine and Human Primary Dendritic Cells into Robust and Modular Artificial Antigen-Presenting Systems by Intracellular Hydrogelation. Adv Mater 33 (30), e2101190.
- Mao, A.S. et al. (2019) Programmable microencapsulation for enhanced mesenchymal stem cell persistence and immunomodulation. Proc Natl Acad Sci U S A 116 (31), 15392-15397.
- Nombela-Arrieta, C. et al. (2011) The elusive nature and function of mesenchymal stem cells. Nat Rev Mol Cell Biol 12 (2), 126-31.
- Sawitzki, B. et al. (2020) Regulatory cell therapy in kidney transplantation (The ONE Study): a harmonised design and analysis of seven non-randomised, single-arm, phase 1/2A trials. The Lancet 395 (10237), 1627-1639.
- Scanzello, C.R. (2017) Role of low-grade inflammation in osteoarthritis. Curr Opin Rheumatol 29 (1), 79-85.
- Sellares, J. et al. (2012) Understanding the causes of kidney transplant failure: the dominant role of antibody-mediated rejection and nonadherence. Am J Transplant 12 (2), 388-99.
- Sousa, A.R. et al. (2021) Engineering Strategies for Allogeneic Solid Tissue Acceptance. Trends Mol Med 27 (6), 572-587.
- Wekerle, T. et al. (2017) Strategies for long-term preservation of kidney graft function. The Lancet 389 (10084), 2152-2162.
- Woodward, K.B. et al. (2020) Pancreatic islets engineered with a FasL protein induce systemic tolerance at the induction phase that evolves into long-term graft-localized immune privilege. Am J Transplant 20 (5), 1285-1295.

## Claims

1. A biomaterial for immunomodulation **characterized in that** it comprises:
- 60% to 100% of aggregates of phenotypically stabilized cells or parts thereof, in dimension ranges of 100 µm to 1 m; and
- 0% to 40% of added biomaterial components, in dimension ranges of 1 µm to 500 µm;
wherein the added biomaterial components are selected from the group consisting of cell-derived materials and exogenously provided materials selected from the group consisting of natural polymers, ceramics, glasses and synthetic polymers;
wherein the cells or parts thereof are natural, engineered, or primed eukaryotic or non-eukaryotic cells or parts thereof or combinations of eukaryotic cells, non-eukaryotic cells or parts thereof, provided that the eukaryotic cells, non-eukaryotic cells or parts thereof have immunomodulatory activity; and
wherein the added biomaterial components and / or the aggregates of phenotypically stabilized cells or parts thereof are joined together through physical forces and / or covalent or non-covalent chemical bonds.

2. The biomaterial for immunomodulation, according to claim 1, **characterized in that** it has a reconfigurable shape configured to allow the biomaterial to adapt to defects with complex shapes, the shape being selected from the group consisting of spheres, fibers, polyhedrons, cell-sheets, bioinks, cylinders, foams, hydrogels, sponges, reticulated cell fragments obtained through disintegration of phenotypically stabilized cell constructs, cream, ointments, patches and adhesives.

3. Biomaterial for immunomodulation, according to claim 1 or 2, **characterized in that** the added biomaterial components include biomaterial particles that have a shape selected from spherical or polyhedral or combinations thereof and the cell aggregates are shaped polyhedrons selected from cell spheroids, cell fibers, cell monolayers, or cells multilayers, wherein the cell spheroids comprise spherical cell aggregates with 50 to 2000 µm diameter, prepared using 10 to 1 million cells, and fiber-shaped aggregates comprises cells deposited in any direction, with length from 200 µm to 1 m.

4. Biomaterial for immunomodulation, according to claim 1, **characterized in that** the eukaryotic cells or parts thereof are selected from the group consisting of mesenchymal stem cells, chimeric-antigen receptor T cells (CAR T-cells), regulatory T cells (Tregs), dendritic cells, macrophages, lymphocytes such as T lymphocytes, B lymphocytes, NK lymphocytes, NKT lymphocytes, cells from adaptive and innate immune system, non-hematopoietic cells that crosstalk with immune system selected from the group consisting of hepatocytes, hepatic stellate cells, liver sinusoidal endothelial cells, and combinations thereof.

5. Biomaterial for immunomodulation, according to claim 1, **characterized in that** the non-eukaryotic cell or parts thereof are selected from the group consisting of bacteria, cyanobacteria, and archaea.

6. Biomaterial for immunomodulation, according to any of the preceding claims, **characterized in that** it further comprises at least one immunomodulatory molecule selected from the group consisting of proteins or glycosaminoglycans from cells or cell-derived components as vesicles or the extracellular matrix, an antigen, an engineered antigen, an antigen presented in the context of major histocompatibility complex molecules or engineered forms thereof, MHC/HLA molecules or engineered forms thereof, co-stimulation molecules, cluster of differentiation 80/86 (CD80/CD86), Fas ligand (Fas-L), programmed death-ligand 1 (PD-L1), HLA class I (HLA-I), HLA-II, CTLA-4 inhibitors, PD1/PD-L1 inhibitors, Fas inhibitors, the clusters of differentiation CD3 or CD28, co-stimulation blockade molecules; immune checkpoint inhibitors, soluble or engineered soluble molecules; transmembrane or engineered transmembrane molecules and membrane or engineered membrane molecules and combinations thereof.

7. Biomaterial for immunomodulation, according to claim 7, **characterized in that** the immunomodulatory molecule is naturally present in the biomaterial deriving from the cell aggregate; or it is artificially added to the biomaterial using non-covalent or covalent bonds; or it is present in the biomaterial due to the previous stimulation of cells through their artificial exposure to immunomodulatory, chemical, and physical factors or genetic engineering tools.

8. Process for production of the biomaterial for immunomodulation as disclosed in any of claims 1 to 7, **characterized in that** it comprises the steps of:
a) tailoring the cells to the desired phenotype;
b) adjusting culture conditions to promote the formation of the cells aggregates; and
c) phenotypically stabilizing the aggregates.

9. Process, according to claim 8, **characterized in that**, in step (a), the cells are incubated and exposed to immunomodulatory, chemical, and physical factors and / or exposed to immunomodulatory agents selected from the group consisting of pro- or anti-inflammatory cytokines.

10. Process, according to claim 8, **characterized in that**, in step (b), the cells are aggregated while in incubation in cell culture medium supplemented with fetal bovine serum or xenogeneic-free factors and antibiotic / antimycotic solution; or while in incubation in cell culture medium supplemented with immunomodulatory agents, added biomaterial components, chemical, or physical factors.

11. Process, according to claim 8, **characterized in that**, in step (c), the stabilization of the cell aggregates is performed by the inhibition of the cellular metabolic activity or the turnover of molecules, by enucleation, or by cellular devitalization, wherein the cell aggregates are immersed in an organic solvent for an exposure period that varies from 1 minute to 96 hours and a temperature range that varies from 4 °C to 120 °C, the organic solvent being selected from the group consisting of ethanol, methanol, dichloromethane, tetrahydrofuran and chloroform.

12. Process, according to claim 11, **characterized in that** the stabilization step is performed by immersing the cell aggregates in fixating agents selected from the group consisting of formaldehyde, paraformaldehyde, glutaraldehyde, genipin, EDC / NHS.

13. Process, according to any of claims 11 and 12, **characterized in that** the stabilization step is performed by a method that is selected from the group consisting of incubation in hyper- or hypotonic saline solutions, incubation in high acidic or alkaline solutions, hydrogelation, lyophilization, supercritical drying, critical point drying, and combinations thereof.

14. Biomaterial for immunomodulation as disclosed in any of claims 1 to 7, **characterized in that** it is for use as an autologous, allogeneic or xenogeneic approach in the treatment, diagnosis and / or prevention of complications in acute and chronic inflammatory diseases or conditions, immune-mediated diseases or conditions, transplantation and post-transplantation of allogeneic grafts or full organs, regenerative medicine, tumors and infections caused by pathogens such as bacteria, mycobacteria or virus.

15. Biomaterial for immunomodulation, according to claim 14, **characterized in that** it is further used synergistically with anti-inflammatory drugs or immunosuppressant drugs.
